(19) Europäisches Patentamt
European Patent Office
Office européen des brevets

(11) **EP 1 021 449 B1**

(12) **FASCICULE DE BREVET EUROPEEN**

(45) Date de publication et mention de la délivrance du brevet:
**02.01.2002 Bulletin 2002/01**

(21) Numéro de dépôt: **98946523.2**

(22) Date de dépôt: **29.09.1998**

(51) Int Cl.7: **C07D 495/04**, C07C 309/73, C07C 309/66
// (C07D495/04, 333:00), C07D221:00

(86) Numéro de dépôt international:
**PCT/FR98/02082**

(87) Numéro de publication internationale:
**WO 99/18110 (15.04.1999 Gazette 1999/15)**

(54) **DERIVES D'ESTERS HYDROXYACETIQUES, LEUR PROCEDE DE PREPARATION ET LEUR UTILISATION COMME INTERMEDIAIRES DE SYNTHESE**

HYDROXYESSIGSÄUREESTER-DERIVATE, VERFAHREN ZU IHRER HERSTELLUNG UND IHRE VERWENDUNG ALS SYNTHESEBAUSTEINE

HYDROXYACETIC ESTER DERIVATIVES, PREPARATION METHOD AND USE AS SYNTHESIS INTERMEDIATES

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **06.10.1997 FR 9712441**

(43) Date de publication de la demande:
**26.07.2000 Bulletin 2000/30**

(73) Titulaire: **SANOFI-SYNTHELABO**
**75013 Paris (FR)**

(72) Inventeurs:
- **BOUSQUET, André**
  **F-04200 Sisteron (FR)**
- **MUSOLINO, Andrée**
  **F-04290 Volonne (FR)**

(74) Mandataire: **Thouret-Lemaitre, Elisabeth et al**
**Sanofi-Synthélabo Service Brevets 174, avenue de France**
**75013 Paris (FR)**

(56) Documents cités:
**EP-A- 0 099 802**
**EP-A- 0 420 706**
**EP-A- 0 465 358**
**EP-A- 0 466 569**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention se rapporte, d'une manière générale, à de nouveaux dérivés d'esters hydroxyacétiques ainsi qu'à leur utilisation comme intermédiaires de synthèse.

Plus précisément, l'invention a pour objet les esters sulfonyloxyacétiques de formule générale:

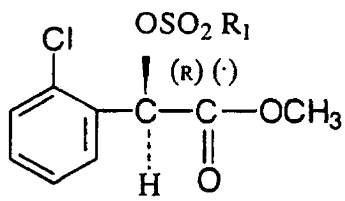

I

dans laquelle $R_1$ représente un groupement benzyle, alkyle en $C_1$ - $C_4$ éventuellement substitué par un ou plusieurs atomes d'halogène tels que chlore ou brome ou un groupement phényle éventuellement substitué par un ou plusieurs atomes d'halogène, par un ou plusieurs groupements alkyles, linéaires ou ramifiés, en $C_1$ - $C_4$ ou par un groupement nitro.

En particulier, l'invention concerne les composés de formule I dans laquelle $R_1$ représente un groupement méthyle, éthyle, propyle, trifluorométhyle, benzyle, phényle, chlorophényle, tolyle, triméthylphényle, triisopropylphényle, dichlorophényle en particulier 2,5-dichlorophényle ou nitrophényle en particulier p-nitrophényle.

Les composés de formule I se sont révélés particulièrement utiles comme produits intermédiaires notamment pour la synthèse du (S)-2-(2-chlorophényl)-2-(4,5,6,7-tétrahydrothiéno [3,2-c]-5-pyridyl)-acétate de méthyle ou clopidogrel.

Cet énantiomère de formule développée:

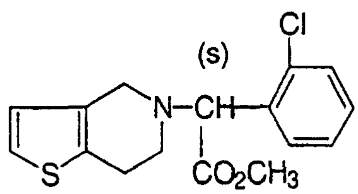

est connu pour son intérêt en thérapeutique notamment pour ses propriétés antiagrégantes plaquettaires et antithrombotiques.

On a décrit dans le brevet EP 0465358 un procédé pour la préparation des énantiomères (R) ou (S) d'un 2-(halogénophényl)-2-(4,5,6,7-tétrahydrothiéno [3,2-c]-5-pyridyl)-acétate d'alkyle en $C_1$ - $C_4$ mettant en oeuvre des esters 2-arylacétiques dotés d'un groupement labile en position 2.

Selon ce procédé:

a) on couple un 2-(halogénophényl)-2-(halogéno-ou alkylsulfonyloxy en $C_1$ - $C_4$ ou arylsulfonyloxy en $C_6$ - $C_{10}$)-acétate d'alkyle en $C_1$ - $C_4$ racémique avec la 4,5,6,7-tétrahydrothiéno [3,2-c] pyridine sous forme de base ou de sel pour obtenir un composé racémique,

b) on résout le racémique ainsi formé par recristallisation de sels d'acides optiquement actifs pour obtenir les énantiomères (R) ou (S) souhaités.

Toutefois, ce procédé n'a été exemplifié qu'au départ du 2-(2-chlorophényl)-2-chloroacétate de méthyle racémique pour la préparation finale du clopidogrel alors qu'aucune indication précise ni aucun exemple ne figure illustrant la préparation d'un dérivé 2-(halophényl)-2-(bromo ou alkylsulfonyloxy ou arylsulfonyloxy)-acétate d'alkyle en $C_1$ - $C_4$.

Selon cet exemple, on obtient le clopidogrel par mise en oeuvre des 5 étapes suivantes au départ d'un ester 2-hydroxyacétique:

a) et b) réaction de l'acide 2-(2-chlorophényl)-2-hydroxyacétique racémique avec le pentachlorure de phosphore

et estérification par le méthanol pour former le 2-(2-chlorophényl)-2-chloroacétate de méthyle racémique avec un rendement de 45%,

c) couplage du 2-(2-chlorophényl)-2-chloroacétate de méthyle ainsi formé, avec la 4,5,6,7-tétrahydrothiéno [3,2-c] pyridine en présence de carbonate de potassium, ce qui fournit le 2-(4,5,6,7-tétrahydrothiéno [3,2-c]-5-pyridyl)-2-(2-chlorophényl)-acétate de méthyle racémique selon un rendement moyen de 80%,

d) résolution du racémique obtenu par salification avec l'acide l-camphosulfonique (rendement: 88 % en sel désiré),

e) régénération du clopidogrel sous forme basique par traitement du sel camphosulfonique en question avec le bicarbonate de sodium.

Selon ce procédé, on recueille le clopidogrel avec un rendement chimique de 30 % maximum à partir de l'acide 2-(2-chlorophényl)-2-hydroxyacétique.

Lors d'essais préliminaires effectués dans le cadre de la présente invention, on a tenté de préparer le clopidogrel ou son énantiomère (R) par mise en oeuvre d'une réaction analogue à celle décrite dans le brevet cité précédemment mais au départ d'énantiomère (R) ou (S) du 2-(2-chlorophényl)-2-chloroacétate de méthyle.

Cependant, tous les essais pratiqués dans le méthanol, l'acétonitrile ou l'acétate d'éthyle comme solvant, à une température comprise entre la température ambiante et 65°C, avec 1 ou 2 équivalents de 4,5,6,7-tétrahydrothiéno [3,2-c] pyridine soit sous forme basique soit sous forme de chlorydrate et en présence ou non de bicarbonate de sodium, ont conduit à la production de 72 à 88 % de 2-(2-chloro-phényl)-2-(4,5,6,7-tétrahydrothiéno [3,2-c]-5-pyridyl)-acétate de méthyle racémique.

D'autres essais pratiqués par chauffage à 80°C avec le (S)-2-(2-chlorophényl)-2-chloroacétate de méthyle et la 4,5,6,7-tétrahydrothiéno [3,2-c] pyridine en présence de carbonate de sodium et dans un mélange de méthylisobutylcétone/eau comme solvant a fourni le (R)-2-(4,5,6,7-tétrahydrothiéno [3,2-c]-5-pyridyl)-2-(2-chlorophényl)-acétate de méthyle avec un excès énantiomérique de 20 % seulement.

Par conséquent, la mise au point d'un procédé pour la préparation du clopidogrel à partir de la 4,5,6,7-tétrahydrothiéno [3,2-c]pyridine selon une méthode stéréosélective comportant un minimum d'étapes et fournissant un rendement appréciable en composé désiré reste d'un intérêt incontestable.

Or, on a découvert de manière surprenante, selon l'invention, que le clopidogrel peut être obtenu en 3 étapes seulement à partir de l'acide (R)-2-(2-chlorophényl)-2-hydroxyacétique selon un rendement global de l'ordre de 80 % tout en faisant intervenir non pas un (R)-2-(2-chlorophényl)-2-halogénoacétate de méthyle mais le (R)-2-(2-chlorophényl)-acétate de méthyle doté d'un groupement sulfonyloxy en position 2, c'est-à-dire un composé de formule I ci-dessus.

Ce procédé est d'autant plus inattendu:

A) que les groupements 2-méthanesulfonyloxy et 2-toluènesulfonyloxy d'esters carboxyliques sont connus pour subir une racémisation lorsqu'ils sont engagés dans une substitution nucléophile par une fonction amine (Angew. Chem. Int. Ed. Eng. 22 (1983), N°1 pp 65 -66).

Ces allégations ont été confirmées dans Tetrahedron, Vol 47, N°7, pp 1109-1135 (1991) où l'on rapporte que les esters 2-méthanesulfonyloxy et 2-p-toluènesulfonyloxy d'acides carboxyliques constituent des substrats inappropriés pour une réaction stéréosélective de substitution nucléophile en position 2.

Des observations analogues ont été publiées dans Liebigs. Ann. Chem. 1986, pp 314-333 puisqu'on y mentionne des rendements < 30 % obtenus lors de la substitution du 2-p-toluènesulfonyloxy- ou 2-méthanesulfonyloxypropionate de méthyle par la benzylamine.

B) que les énantiomères (R) ou (S) d'esters 2-sulfonyloxyacétiques comportant en outre un groupement 2-phényle sont reconnus comme fournissant, après substitution nucléophile en position 2, des composés avec stéréosélectivité très réduite.

Par exemple, on a décrit dans Tetrahedron, Vol 44, N°17, pp 5583-5595 (1988), la substitution de dérivés (S) ou (R)-2-triflyloxy-2-X-acétate de méthyle par la O-benzylhydroxylamine dans le dichlorométhane comme solvant et à une température comprise entre 0°C et la température ambiante de manière à former les esters respectivement (R) ou (S)-2-O-benzylhydroxylamino-2-X-acétiques. Comme il est connu, le groupe triflyle désigne le radical trifluorométhylsulfonyle.

Si cette réaction s'est avérée hautement sélective dans le cas des esters où X représente un groupement alkyle éventuellement substitué tel que méthyle ou benzyle (excès énantiomérique ee: ≥ 95 %), par contre, cette sélectivité s'est révélée fortement diminuée lorsque X représente le groupement phényle puisque l'excès énantiomérique en ester (R)-2-O-benzylhydroxylamino-2-phényle correspondant n'est plus que de 50%. Des résultats tout à fait analogues ont été rapportés à la suite d'autres expérimentations pratiquées avec des dérivés 2-sulfonyloxy d'esters 2-phénylacétiques.

Ainsi:

a) Tetrahedron Letters, Vol 31, n° 21, pp 2953-2956 (1990) décrit la réaction de substitution des esters (S)-2-triflyloxy-2-X-acétate de méthyle par la t-butyloxycarbonylhydrazine ou BOC-hydrazine, dans le dichlorométhane et à 0°C, de manière à former des esters (R)-2-BOC-hydrazinyl-2-X-acétiques.

La sélectivité de cette réaction s'est également révélée très importante lorsque X représente un groupement alkyle éventuellement substitué, par exemple méthyle, isobutyle ou benzyle (ee: >95%) mais par contre très pauvre lorsque X représente phényle (ee: 28 %)

b) Tetrahedron, Vol 48, N° 15, pp 3007-3020 (1992) rapporte la substitution de dérivés (S)-2-nosyloxy-2-X-acétate de méthyle par le groupement azido, la réaction ayant lieu dans le dichlorométhane et à température ambiante, de manière à former des esters (R)-2-azido-2-X-acétiques.

A nouveau, la substitution du groupement 2-sulfonyloxy, dans ce cas le groupement nosyloxy, s'est opérée de manière hautement sélective lorsque X représente un groupement alkyle éventuellement substitué par exemple méthyle, isopropyle, sec-butyle, isobutyle ou benzyle (ee: > 92 %) mais décevante lorsque X représente le groupement phényle (ee: 35 %).

Ces derniers résultats ont d'ailleurs amené les auteurs de la publication en question à conclure que "le groupement phényle est bien connu pour diminuer la stéréosélectivité lors de la substitution des esters 2-nosyloxy et 2-triflyloxy par différentes classes d'agents nucléophiles". Comme il est connu, le groupe nosyle désigne le radical p-nitrophénylsulfonyle.

Les dérivés sulfonyloxy d'esters acétiques de formule I, qui se sont montrés particulièrement intéressants comme intermédiaires de synthèse notamment pour la préparation du clopidogrel, sont:

- le (R)-2-benzènesulfonyloxy-2-(2-chlorophényl)-acétate de méthyle
- le (R)-2-(2-chlorophényl)-2-(p-toluènesulfonyloxy)-acétate de méthyle
- le (R)-2-(2-chlorophényl)-2-méthanesulfonyloxy-acétate de méthyle
- le (R)-2-(4-chlorobenzènesulfonyloxyl)-2-(2-chlorophényl)-acétate de méthyle
- le (R)-2-(2-chlorophényl)-2-(2,4,6-triméthylbenzènesulfonyloxy)-acétate de méthyle
- le (R)-2-(2-chlorophényl)-2-(2,4,6-trisopropylbenzènesulfonyloxy)-acétate de méthyle
- le (R)-2-(2-chlorophényl)-2-(4-nitrobenzènesulfonyloxy)-acétate de méthyle.
- le (R)-2-(2-chlorophényl)-2-(2,5-dichlorobenzènesulfonyloxy)-acétate de méthyle

En particulier, on préfère les:

- (R)-2-benzènesulfonyloxy-2-(2-chlorophényl)-acétate de méthyle
- (R)-2-(2-chlorophényl)-2-(2,4,6-triméthylbenzènesulfonyloxy)-acétate de méthyle
- (R)-2-(2-chlorophényl)-2-(2,4,6-trisopropylbenzènesulfonyloxy)-acétate de méthyle
- (R)-2-(2-chlorophényl)-2-(4-nitrobenzènesulfonyloxy)-acétate de méthyle
- (R)-2-(4-chlorobenzènesulfonyloxy)-2-(2-chlorophényl)-acétate de méthyle
- (R)-2-(2-chlorophényl)-2-(2,5-dichlorobenzènesulfonyloxy)-acétate de méthyle.

Les dérivés sulfonyloxy de l'invention peuvent être obtenus par réaction de l'ester (R)-2-(2-chlorophényl)-2-hydroxy-acétique de formule:

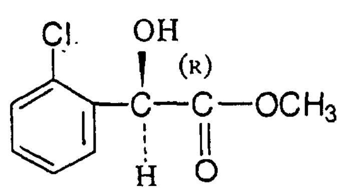

II

avec un anhydride ou halogénure de sulfonyle de formule générale:

$$R_1 - SO_2 - R_2 \qquad III$$

dans laquelle $R_2$ représente un groupement - $OSO_2$ - $R_1$ ou, de préférence, un atome d'halogène tel que chlore ou

brome et $R_1$ a la même signification que précédemment, en présence d'un sel de lithium et d'une amine aromatique fonctionnant à la fois comme catalyseur et accepteur d'acide telle que la pyridine ou la 4-diméthylaminopyridine, ce qui fournit le composé désiré.

La réaction en question est généralement conduite dans un solvant aprotique tel qu'un hydrocarbure aliphatique halogéné de préférence en $C_1$-$C_4$, par exemple le dichlorométhane, le dichloroéthane, le chloroforme, le tétrachlorure de carbone ou encore le tétrachloréthane et à une température comprise entre 0°C et la température ambiante.

En outre, l'amine aromatique de préférence en $C_6$-$C_{10}$, est utilisée en quantité stoechiométrique ou de préférence en léger excès pouvant atteindre 1,2 équivalent molaire par rapport au composé de formule III.

La réaction de couplage des composés de formule II et formule III dans laquelle $R_2$ représente un atome d'halogène, peut donner naissance à une réaction secondaire à l'origine de la formation d'un dérivé halogéné à savoir un dérivé 2-(2-chlorophényl)-2-halogénoacétate de méthyle.

Or, on a remarqué de manière fortuite que la présence d'un sel de lithium dans le milieu réactionnel permet de minimiser fortement cette réaction secondaire et par voie de conséquence de fournir des taux importants en composé de formule I.

A titre d'exemple, la réaction entre le (R)-2-(2-chlorophényl)-2-hydroxyacétate de méthyle avec le chlorure de p-nitrobenzènesulfonyle ou le chlorure de p-toluènesulfonyle dans le dichlorométhane à 20°C et en présence de pyridine et de perchlorate de lithium fournit après 5 heures, respectivement le (R)-2-(2-chloro-phényl)-2-(p-nitrobenzènesulfonyloxy)-acétate de méthyle avec un rendement de 92 % et le (R)-(2-chlorophényl)-2-(p-toluènesulfonyloxy)-acétate de méthyle avec un rendement de 85 % alors que cette même réaction en l'absence de perchlorate de lithium rie produit que respectivement 28 % et 30 % en produit souhaité.

Ce sel de lithium, qui peut être par exemple le perchlorate de lithium (Li $ClO_4$) ou le tétrafluoroborate de lithium (Li $BF_4$), est utilisé en quantité stoechiométrique. Toutefois, on préfère mettre en oeuvre ce sel de lithium en léger excès c'est-à-dire jusqu'à 1,2 équivalent molaire par rapport au composé de formule III.

Quant à l'ester de formule II, on peut l'obtenir selon une réaction non racémisante en estérifiant l'acide (R)-2-(2-chlorophényl)-2-hydroxyacétique ou acide (R)-2-chloro-mandélique, qui est un produit commercial, avec le méthanol et en présence d'un acide fort tel que l'acide sulfurique.

Selon une variante du procédé ci-dessus, on peut également préparer les dérivés sulfonyloxy de formule I en faisant réagir l'ester de formule II avec un halogénure de formule III en présence de 4-diméthylaminopyridine comme catalyseur et d'un autre accepteur d'acide tel qu'une amine aliphatique par exemple la triéthylamine pour fournir le composé souhaité.

La réaction se déroule en général à une température de - 10°C à + 10°C préférentiellement à 0°C et dans un solvant aprotique tel que ceux évoqués précédemment notamment le dichlorométhane.

Les procédés décrits ci-dessus permettent d'obtenir les dérivés sulfonyloxy de formule I selon des rendements chimiques particulièrement intéressants, habituellement de l'ordre de 90 à 98 % et avec des excès énantiomériques tout à fait remarquables puisque > 99 %.

Comme indiqué précédemment, les dérivés sulfonyloxy de l'invention peuvent être utilisés notamment pour la préparation du clopidogrel.

En conséquence, un autre objet de l'invention se rapporte à la préparation du clopidogrel selon un procédé par lequel on fait réagir la 4,5,6,7-tétrahydrothiéno [3,2-c] pyridine sous forme de base ou de sel avec un dérivé sulfonyloxy de formule I en présence d'un agent basique utilisé seul ou en solution aqueuse, ce qui fournit le composé souhaité.

Comme milieu réactionnel, on utilise habituellement un solvant polaire tel qu'un ester aliphatique en $C_2$ - $C_5$ par exemple l'acétate d'éthyle ou d'isopropyle, un alcool aliphatique en $C_1$ - $C_4$, le N,N-diméthylformamide, un éther cyclique en $C_4$-$C_6$ ou aliphatique en $C_2$-$C_6$ tels que le tétrahydrofuranne ou l'éther isopropylique, une cétone aliphatique en $C_2$-$C_8$ par exemple la méthylisobutylcétone ou, de préférence, un solvant non polaire tel qu'un hydrocarbure aliphatique halogéné en $C_1$-$C_4$ par exemple le dichlorométhane, le dichloroéthane, le chloroforme, le tétrachlorure de carbone ou le tétrachloroéthane ou encore un hydrocarbure aromatique en $C_6$-$C_{10}$ par exemple le benzène, le toluène ou un xylène de manière à former un système biphasique lorsque l'eau est présente dans le milieu réactionnel. Dans ce dernier cas, on peut utiliser, si nécessaire un catalyseur de transfert de phase tel qu'un ammonium quaternaire, un sel de phosphonium ou un éther couronne.

De même, l'agent basique peut être un carbonate de métal alcalin tel que le carbonate de sodium ou de potassium ou un bicarbonate de métal alcalin par exemple le bicarbonate de sodium ou de potassium tandis que la réaction peut être entreprise à une température pouvant varier de la température ambiante à la température de reflux du milieu utilisé.

Quant à la 4,5,6,7-tétrahydrothiéno [3,2-c] pyridine, produit connu, on l'utilise en quantité stoechiométrique mais habituellement et préférentiellement en excès pouvant atteindre 2,5 équivalents molaires par rapport au dérivé sulfonyloxy de formule I.

Par mise en oeuvre de cette dernière réaction dans le toluène, la méthylisobutylcétone ou l'acétate d'isopropyle à la température de 80°C en environ 4 heures, on peut recueillir le clopidogrel selon un rendement chimique supérieur à 95 % et avec un excès énantiomérique compris entre 80 et 88 %.

Dans le dichlorométhane comme solvant et à la température de 40°C :

a) le taux de conversion en clopidogrel peut atteindre 94 à 95% en 5 heures avec un excès énantiomérique de 96%.
b) la conversion en clopidogrel est totale en 10 heures à partir du (R)-2-(4-chlorobenzènesulfonyloxy)-2-(2-chlorophényl)-acétate de méthyle et l'excès énantiomérique est de 96%.
c) la conversion en clopidogrel est totale en 30 minutes à partir du (R)-2-(2-chlorophényl)-2-(4-nitrobenzènesulfonyloxy)-acétate de méthyle et l'excès énantiomérique > 98%.

Selon une voie alternative, on peut également préparer le clopidogrel à partir des dérivés sulfonyloxy de l'invention et de la 2-(thién-2-yl)éthylamine.

En conséquence, l'invention se rapporte également à la préparation du clopidogrel selon un procédé par lequel :

a) on fait réagir la 2-(thién-2-yl)éthylamine sous forme de base ou de sel avec un dérivé sulfonyloxy de formule I en présence d'un agent basique utilisé seul ou en solution aqueuse, pour former le (+)-(S)-α-(2-(thién-2-yl)éthylamino)-α-(2-chlorophényl)-acétate de méthyle de formule:

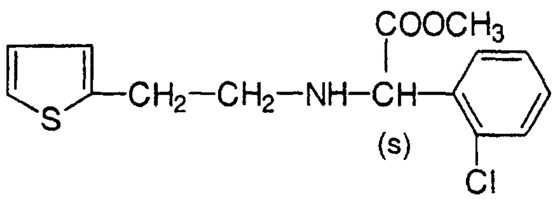

IV

b) on fait réagir le dérivé de thiényléthylamine ainsi formé, avec un agent de formylation et on cyclise en présence d'un acide, ce qui fournit le composé souhaité.

L'agent de formylation utilisé dans le procédé ci-dessus peut être:

- soit l'aldéhyde formique ou tout composé généralement connu pour le libérer sous forme réactive tel que par exemple son hydrate ou ses dérivés de polymérisation. Ces agents de formylation peuvent être considérés comme préférés dans le cadre de l'invention.
- soit des composés de formule générale :

$$R_3 - CH_2 - R_4 \qquad V$$

dans laquelle $R_3$ représente un atome d'halogène, un groupement alkoxy en $C_1$ -$C_4$, un groupement alkylthio en $C_1$ -$C_4$ ou un groupement amino et $R_4$ représente un groupement alkoxy en $C_1$ -$C_4$, alkylthio en $C_1$ -$C_4$, amino, alkoxycarbonyle en $C_2$ -$C_5$ ou phénoxycarbonyle
- soit des composés hétérocycliques de formule générale:

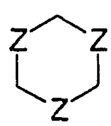

dans laquelle Z représente O, NH ou S, tels que le s-trioxane.

L'étape impliquant l'utilisation de la 2-(thién-2-yl)éthylamine, produit connu, peut être entreprise selon les

mêmes conditions opératoires que celles énoncées précédemment pour la mise en oeuvre de la 4,5,6,7-tétrahydrothiéno [3,2-c] pyridine.

L'étape de formylation et de cyclisation, quant à elle peut, peut engendrer des composés intermédiaires tels qu'une hydroxyméthylèneamine ou un hétérocycle de type triméthylènetriamine, comme il est rapporté dans le brevet EP 0 466 569.

En conséquence, la réaction avec l'agent de formylation et la cyclisation peuvent se dérouler de manière successive, éventuellement en isolant les composés intermédiaires en question, ou au contraire, de manière simultanée.

Lorsque les réactions sont successives, l'étape impliquant l'agent de formylation peut être entreprise éventuellement en présence d'un éther, d'un solvant hydrocarboné tel que par exemple le benzène, le toluène, un xylène ou l'éther de pétrole ou d'un solvant halogéné tel que le chlorure de méthylène ou le dichloroéthane.

La cyclisation est ensuite effectuée dans un solvant polaire tel que l'eau, un alcool, le diméthylformamide ou dans un mélange de ces solvants.

Comme agent de formylation, on préfère généralement l'aldéhyde formique qui peut être introduit sous forme de solution aqueuse dans le milieu réactionnel.

Quant à l'acide, il peut s'agir d'un acide organique ou minéral en général un acide fort tel que l'acide sulfurique ou un hydracide tel que l'acide chlorhydrique ou encore un acide sulfonique tel que l'acide méthanesulfonique.

Lorsque les réactions sont effectuées simultanément, le milieu réactionnel est formé d'un solvant polaire tel que l'eau ou un alcool et l'acide, qui peut être minéral ou organique, est introduit dans le milieu, de préférence en quantité stoechiométrique par rapport au composé de formule IV mis en oeuvre. Dans ce cas, cet acide est un acide fort qui peut être simplement introduit dans le milieu sous forme de son sel avec le composé de formule IV. On peut aussi utiliser un solvant acide tel que l'acide formique ou l'acide acétique, le premier associé au paraformaldéhyde étant particulièrement préféré.

Le clopidogrel obtenu selon l'invention, c'est-à-dire par mise en oeuvre des différentes méthodes ci-dessus, peut alors être purifié si nécessaire, de manière classique par un procédé de recristallisation ou par des techniques chromatographiques.

Les exemples suivants illustrent l'invention:

PREPARATION

(R)-2-hydroxy-2-(2-chlorophényl)-acétate de méthyle

Dans un réacteur de 1000 ml muni d'une double enveloppe et d'une vanne de fond, d'un agitateur mécanique, d'un thermomètre et d'un réfrigérant, on charge 120 g (0,643 mole) d'acide (R)-2-hydroxy-2-(2-chlorophényl)-acétique, 480 ml de méthanol et 4,8 g d'acide sulfurique à 95 %. On chauffe alors à reflux pendant 2 heures la solution obtenue puis on élimine l'excès de méthanol sous pression réduite. On reprend le résidu huileux dans 650 ml de dichlorométhane et 240 g d'une solution aqueuse de carbonate de potassium à 10 %.

Après décantation, on lave la phase chlorée avec 200 ml d'eau puis on concentre sous pression réduite.

De cette manière on obtient 124,4 g de (R)-2-hydroxy-2-(2-chlorophényl)-acétate de méthyle sous forme d'une huile incolore.

Rendement: 94 %.
Pureté optique par chromatographie liquide sur phase chirale: > 99 %.
RMN ($CDCl_3$):

7,4-7,2 ppm (multiplet; 4 protons aromatiques)
5,57 ppm (singulet, 1 proton CH)
3,76 ppm (singulet, 3 protons $OCH_3$)
3,59 ppm (singulet large, 1 proton OH)

EXEMPLES 1 à 7

(R)-2-Benzènesulfonyloxy-2-(2-chlorophényl)-acétate de méthyle

(exemple 1)

Dans un ballon tricol sec de 100 ml, muni d'un agitateur magnétique, d'un réfrigérant et d'un thermomètre, on charge , sous atmosphère d'azote, 3,81 g (36 mmoles) de perchlorate de lithium, 30 mmoles de chlorure de benzènesulfonyle et 45 ml de dichloroéthane.

A la solution obtenue, on ajoute 2,9 ml (36 mmoles) de pyridine. On agite alors pendant 15 minutes le milieu réactionnel blanc hétérogène puis on y introduit 6,0 g de (R)-2-hydroxy-2-(2-chlorophényl)-acétate de méthyle préalablement solubilisé dans 15 ml de dichloroéthane.

On maintient sous agitation pendant 5 heures le milieu laiteux obtenu puis on le verse sur un mélange placé sous agitation et composé de 120 ml d'acide chlorhydrique 1 N et 240 ml de dichlorométhane. Après décantation, on lave la phase chlorée avec 120 ml d'eau puis on concentre sous pression réduite. Le sulfonate ainsi formé se présente sous forme d'un liquide visqueux incolore. Une purification sur colonne de silice permet l'obtention d'un échantillon analytiquement pur.

De cette manière, on obtient le (R)-2-benzènesulfonyloxy-2-(2-chlorophényl)-acétate de méthyle.

Rendement: 90 %
Pureté optique: > 99 %
$[\alpha]_{589}^{25}$ : - 53° (2%, méthanol)
RMN ($CDCl_3$):

7,88 ppm (doublet de triplets, 2 protons aromatiques)
7,63 à 7,55 ppm (multiplet, 1 proton aromatique)
7,50 à 7,38 ppm (multiplet, 3 protons aromatiques)
7,33 à 7,17 ppm (multiplet, 3 protons aromatiques)
6,30 ppm (singulet, 1 proton (CH))
3,70 ppm (singulet, 1 proton ($OCH_3$))

En suivant le même procédé que celui décrit précédemment, on a préparé les composés suivants:

<u>(R)-2-(2-chlorophényl)-2-(4-p-toluènesulfonyloxy)-acétate de méthyle</u>

(Exemple 2)

Rendement: 85 %
Pureté optique: > 99 %
$[\alpha]_{589}^{25}$ : - 58,3° (2%, méthanol)
RMN ($CDCl_3$):

7,75 à 7,25 ppm (multiplets, 8 protons aromatiques)
5,79 ppm (singulet, 1 proton (CH - O))
3,67 ppm (singulet, 3 protons ($OCH_3$))
2,41 ppm (singulet, 3 protons ($CH_3$ - phényl)

<u>(R)-2-(2-chlorophényl)-2-méthanesulfonyloxy-acétate de méthyle</u>

(Exemple 3)

Rendement: 87 %
Pureté optique: > 99 %
$[\alpha]_{589}^{25}$ : -75,6° (2%, méthanol)
RMN ($CDCl_3$):

7,49 à 7,28 ppm (multiplet, 4 protons aromatiques)
6,40 ppm (singulet, 1 proton (CH))
3,79 ppm (singulet, 3 protons ($OCH_3$)
3,14 ppm (singulet, 3 protons ($SO_2CH_3$)

(R)-2-(4-chlorobenzènesulfonyloxy)-2-(2-chlorophényl)-acétate de méthyle

(Exemple 4)

Rendement: 90 %
Pureté optique: > 99 %
RMN ($CDCl_3$):

7,8 et 7,43 ppm (2 doublets, 4 protons aromatiques)
7,42 à 7,18 ppm (multiplet, 4 protons aromatiques)
6,31 ppm (singulet, 1 proton (CH))
3,73 ppm (singulet, 3 protons ($OCH_3$)

(R)-2-(2-chlorophényl)-2-(2,4,6-triméthylbenzènesulfonyloxy)-acétate de méthyle

(Exemple 5)

Rendement: 93 %
Pureté optique: > 99 %
RMN ($CDCl_3$):

7,50 à 7,20 ppm (multiplet, 4 protons aromatiques)
6,92 ppm (singulet, 2 protons aromatiques)
6,21 ppm (singulet, 1 proton (CH))
3,69 ppm (singulet, 3 protons ($OCH_3$))
2,62 ppm (singulet, 6 protons ($CH_3$))
2,30 ppm (singulet, 3 protons (CH3))

(R)-2-(2-chlorophényl)-2-(2,4,6-triisopropylbenzènesulfonyloxy)-acétate de méthyle

(Exemple 6)

Rendement: 93 %
Pureté optique: > 99 %
RMN ($CDCl_3$):

7,50 à 7,20 ppm (multiplet, 4 protons aromatiques)
7,14 ppm (singulet, 2 protons aromatiques)
6,25 ppm (singulet, 1 proton (CH))
4,09 ppm (septuplet, 2 protons (2 CH isopropyle))
3,71 ppm (singulet, 3 protons ($OCH_3$))
2,85 ppm (septuplet, 1 proton (CH isopropyle)
1,24 ppm (doublet, 6 protons (2 $CH_3$))
1,22 ppm (doublet, 6 protons (2$CH_3$)
1,10 ppm (doublet, 6 protons (2 $CH_3$))

(R)-2-(2-chlorophényl)-2-(4-nitrobenzènesulfonyloxy)-acétate de méthyle

(Exemple 7)

Rendement: 92 %
Pureté optique: > 99 %
RMN ($CDCl_3$):

8,29 et 8,06 ppm (2 doublets, 4 protons aromatiques)
7,40 à 7,15 ppm (multiplet, 4 protons aromatiques)
6,37 ppm (singulet, 1 proton (CH))
3,74 ppm (singulet, 3 protons ($OCH_3$)

EXEMPLE 8

(R)-2-Benzènesulfonyloxy-2-(2-chlorophényl)-acétate de méthyle

Dans un ballon tricol sec muni d'une double enveloppe, équipé d'un agitateur magnétique, d'un réfrigérant et d'un thermomètre, on charge, sous atmosphère d'azote, 0,72 g (6 mmoles) de 4-diméthylaminopyridine, 12,0 g (60 mmoles) de (R)-2-hydroxy-2-(2-chlorophényl)-acétate de méthyle, 6,06 g (60 mmoles) de triéthylamine et 20 ml de dichlorométhane. On refroidit à 0°C la solution incolore obtenue puis, à cette température, on introduit 60 mmoles de chlorure de benzènesulfonyle en solution dans 30 ml de dichlorométhane. On laisse le milieu réactionnel sous agitation à 0°C durant 3 heures puis on le verse sur un mélange, placé sous agitation, composé de 240 ml d'acide chlorhydrique 1 N et 240 ml de dichlorométhane.

Après décantation, on lave la phase chlorée avec 120 ml d'eau puis on concentre sous pression réduite.

Le sulfonate ainsi formé se présente sous forme d'un liquide visqueux incolore. Une purification sur colonne de silice permet l'obtention d'un échantillon analytiquement pur.

De cette manière, on obtient le (R)-2-benzènesulfonyloxy-2-(2-chlorophényl)-acétate de méthyle.

Rendement: 97 %
Pureté optique: >99 %
$[\alpha]^{25}_{589}$ : - 53° (2%, méthanol)

En suivant le même procédé que celui décrit précédemment, on a obtenu les composés suivants :

(R)-2-(2-chlorophényl)-2-(4-nitrobenzènesulfonyloxy)-acétate de méthyle

(Exemple 9)

Rendement : 88 %
Pureté optique: > 99 %

(R)-2-(2-chlorophényl)-2-(2,5-dichlorobenzènesulfonyloxy)-acétate de méthyle

(Exemple 10)

Rendement : 95%
Pureté optique : > 99%
RMN ($CDCl_3$) :

7,98ppm (doublet, 1 proton aromatique)
7,15 à 7,50ppm (multiplets, 6 protons aromatiques)

6,38ppm (singulet, 1 proton (CH))
3,74ppm (singulet, 3 protons ($OCH_3$))

EXEMPLE 11

(R)-2-Benzènesulfonyloxy-2-(2-chlorophényl)-acétate de méthyle

Ce composé a été obtenu selon la méthode décrite dans l'Exemple 8 mais en remplaçant le dichlorométhane par le toluène.
Rendement: > 95 %

EXEMPLE 12

(R)-2-Benzènesulfonyloxy-2-(2-chlorophényl)-acétate de méthyle

Dans un ballon tricol équipé comme à l'Exemple 8, on charge 0,72 g (6 mmoles; 0,1 équivalent) de 4-diméthylaminopyridine, 12,0 g (60 mmoles; 1 équivalent) de (R)-2-hydroxy-2-(2-chlorophényl)-acétate de méthyle et 7,8 g (78 mmoles; 1,3 équivalent) de triéthylamine et 20 ml de dichlorométhane. On refroidit à 0°C la solution incolore obtenue puis,à cette température, on introduit 6,06 g (60 mmoles; 1 équivalent) de chlorure de benzènesulfonyle en solution dans 30 ml de dichlorométhane. On laisse le milieu réactionnel sous agitation à 0°C durant 3 heures, puis on le verse sur un mélange placé sous agitation, composé de 240 ml d'acide chlorhydrique 1 N et 240 ml de dichlorométhane. Après décantation, on lave la phase chlorée avec de l'acide chlorhydrique dilué puis avec de l'eau ensuite on concentre sous pression réduite. Une purification sur colonne de silice permet l'obtention d'un échantillon analytiquement pur.
De cette manière, on obtient le (R)-2-benzènesulfonyloxy-2-(2-chlorophényl)-acétate de méthyle.

Rendement: 98 %
Pureté optique: 100 % (énantiomère S (+) non détecté).

EXEMPLE 13

(S)-2-(2-chlorophényl)-2-(4,5,6,7-tétrahydrothiéno [3,2-c]-5-pyridyl)-acétate de méthyle

Dans un ballon tricol de 50 ml, muni d'une double enveloppe et équipé d'un agitateur magnétique, d'un réfrigérant et d'un thermomètre, on charge Y mmoles de 4,5,6,7-tétrahydrothiéno [3,2-c] pyridine en solution dans 7,5 ml de solvant et 2,85 g d'une solution aqueuse à 30 % de carbonate de potassium. Après 10 minutes d'agitation, on ajoute 5 mmoles de composé de formule I préalablement dissous dans 2,5 ml de solvant.
On chauffe au reflux, durant le temps indiqué, le milieu biphasique ainsi obtenu, on le refroidit à 70°C puis on le décante.
De cette manière on obtient le (S)-2-(2-chlorophényl)-2-(4,5,6,7-tétrahydrothiéno [3,2-c]-5-pyridyl)-acétate de méthyle ou clopidogrel.

RMN ($CDCl_3$):

7,70 ppm (multiplet; 1 proton aromatique du benzène)
7,41 ppm (multiplet; 1 proton aromatique du benzène)
7,33 à 7,22 ppm (multiplet; 2 protons aromatiques du benzène)
7,06 ppm (doublet; 1 proton aromatique du thiophène)
6,67 ppm (doublet; 1 proton aromatique du thiophène)
4,93 ppm (singulet; 1 proton CHCO)
3,73 ppm(singulet; 3 protons $OCH_3$)
3,76 et 3,64 ppm (2 doublets; 2 protons $CH_2$)
2,89 ppm (singulet; 4 protons $2CH_2$)

Selon les composés de formule I employés, les concentrations en 4,5,6,7 -tétrahydrothiéno [3,2-c] pyridine utilisées, les solvants et les temps de réaction ci-dessus, on a obtenu les rendements suivants:

| Composé de formule I | Y (mmoles) | Solvant | Temps de réactio n (h) | Clopidogrel | |
|---|---|---|---|---|---|
| $R_1$ | | | | Rendement (%) | Pureté optique (%) |
| phényle | 5 (1,7g; 1 équivalent) | toluène | 4,5 | 85 | 90 |
| phényle | 6 (1,2 équivalent) | dichlorométhane | 5 | 94,5 | 96,2 |
| phényle | 12,5 (2,5 équivalent) | dichlorométhane | 5 | 98,5 | 89 |
| 4-chlorophényle | 6 | dichlorométhane | 10 | environ 100 | 96 |
| 4-nitrophényle | 6 | dichlorométhane | 0,5 | environ 100 | > 98 |

EXEMPLE 14

(S)-2-(2-chlorophényl)-2-(4,5,6,7-tétrahydrothiéno[3,2-c]-5-pyridyl)-acétate de méthyle

Dans un ballon tricol de 50 ml muni d'une double enveloppe et équipé d'un agitateur magnétique, d'un réfrigérant et d'un thermomètre, on charge 1,2 mmoles de 4,5,6,7-tétrahydrothiéno [3,2-c]pyridine en solution dans 7,5 ml de dichlorométhane et 2,85g d'une solution aqueuse à 30% de carbonate de potassium. Après 10 minutes d'agitation, on ajoute 5 mmoles de (R)-2-(2-chlorophényl)-2-(2,5-dichlorobenzènesulfonyloxy)-acétate de méthyle préalablement dissous dans 2,5 ml de solvant.

On chauffe au reflux durant 3,5 heures, le milieu biphasique ainsi obtenu, on le refroidit à 70°C puis on le décante.

De cette manière, on obtient le (S)-2-(2-chlorophényl)-2-(4,5,6,7-tétrahydrothiéno[3,2-c]-5-pyridyl-acétate de méthyle.

Rendement : 89 %
Pureté optique : 98 %

EXEMPLE 15

Hémisulfate de (S)-2-(2-chlorophényl)-2-(4,5,6,7-tétrahydrothiéno[3,2-c]-5-pyridyl)-acétate de méthyle ou hémisulfate de clopidogrel

a) (+)-(S)-α-(2-thién-2-yl)éthylamino)-α-(2-chlorophényl)-acétate de méthyle.

Dans un réacteur de 250 ml, muni d'une double enveloppe et équipé d'un moteur d'agitation, d'un réfrigérant et d'un thermomètre, on charge 7,62g de 2-(thién-2-yl)éthylamine(0,06 mole; 1,2 équivalent molaire par rapport au dérivé sulfonyloxy) en solution dans 67,5 ml de dichlorométhane et une solution aqueuse de 7,0g d'hydrogénocarbonate de potassium (0,07 mole; 1,4 équivalent par rapport au dérivé sulfonyloxy) dans 30 ml d'eau. Après 5 minutes d'agitation, on ajoute 0,05 mole (1 équivalent) de composé de formule I préalablement dissous dans 40 ml de dichlorométhane.

On chauffe au reflux le milieu biphasique durant le temps indiqué, on le refroidit puis, on le décante pour recueillir le (+)-(S)-α-(2-thién-2-yl)éthylamino-α-(2-chlorophényl)-acétate de méthyle (formule IV).

Selon les composés de formule I employés et les temps de réaction ci-dessus, on a obtenu les rendements suivants :

| Composé de formule I | Temps de réaction (h) | Composé de formule IV | |
|---|---|---|---|
| R1 | | Rendement (%) | Pureté optique (%) |
| méthyle | 29 | 65 | 76 |

(suite)

| Composé de formule I | Temps de réaction (h) | Composé de formule IV | |
|---|---|---|---|
| R1 | | Rendement (%) | Pureté optique (%) |
| phényle | 22 | 71 | 92 |
| 4-chlorophényle | 11 | 94 | 92 |
| 4-nitrophényle | 2 | 99 | 95 |
| 2,5-dichlorophényle | 6 | 98 | 97 |

b) Hémisulfate de clopidogrel

Sous agitation, on introduit en 25 minutes dans 40 ml d'une solution aqueuse d'aldéhyde formique à 30 % (p/p), 20,5 g de (+)-(S)-α-(2-thién-2-yl)éthylamino)-α-(2-chlorophényl)-acétate de méthyle en solution dans 200 ml de chlorure de méthylène.

Après 3 heures d'agitation, on décante la phase organique, on lave à l'eau, sèche et évapore le solvant. On dissout le résidu dans 50 ml de chlorure de méthylène et on introduit la solution, à 60°C, dans 100 ml de N,N-diméthylformamide anhydre contenant de l'acide chlorhydrique à concentration 6N. Après 1h30 à cette température, on élimine les solvants par distillation sous pression réduite et on dissout le résidu dans 200 ml de chlorure de méthylène et 100 ml d'eau.

On ajoute du bocarbonate de sodium pour libérer la base de son chlorhydrate, décante la phase organique, sèche et concentre sous pression réduite ce qui fournit le clopidogrel sous forme de base libre.

On forme alors l'hémisulfate de- ce produit basique dans 150 ml d'acétone par action de 4,9 g d'acide sulfurique concentré (96%).

De cette manière, on obtient 17 g d'hémisulfate de clopidogrel $[\alpha]_D^{20}$ = + 53° (C = 1,méthanol)

## Revendications

**1.** Dérivés d'esters (R)-sulfonyloxyacétiques de formule générale:

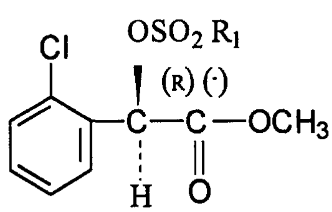

I

dans laquelle $R_1$ représente un groupement benzyle, alkyle en $C_1$ - $C_4$ éventuellement substitué par un ou plusieurs atomes d'halogène, ou phényle éventuellement substitué par un ou plusieurs atomes d'halogène ou par un ou plusieurs groupements alkyles, linéaires ou ramifiés, en $C_1$ -$C_4$ ou par un groupement nitro.

**2.** Dérivés d'esters (R)-sulfonyloxyacétiques selon la Revendication 1, dans laquelle $R_1$ représente un groupement méthyle, éthyle, propyle, trifluorométhyle, benzyle, phényle, chlorophényle, tolyle, triméthylphényle, triisopropylphényle, dichlorophényle ou nitrophényle.

**3.** (R)-2-Benzènesulfonyloxy-2-(2-chlorophényl)-acétate de méthyle.

**4.** (R)-2-(2-chlorophényl)-2-(2,4,6-triméthylbenzènesulfonyloxy)-acétate de méthyle.

**5.** (R)-2-(4-chlorobenzènesulfonyloxy)-2-(2-chlorophényl)-acétate de méthyle.

**6.** (R)-2-(2-chlorophényl)-2-(4-nitrobenzènesulfonyloxy)-acétate de méthyle.

**7.** (R)-2-(2-chlorophényl)-2-(2,4,6-triisopropylbenzènesulfonyloxy)-acétate de méthyle.

**8.** (R)-2-(2-chlorophényl)-2-(2,5-dichlorobenzènesulfonyloxy)-acétate de méthyle.

**9.** Procédé de préparation d'esters (R)-sulfonyloxyacétiques selon une des Revendications 1 à 8, **caractérisé en ce que** l'on fait réagir l'ester (R)-2-(2-chloro-phényl)-2-hydroxy-acétique de formule:

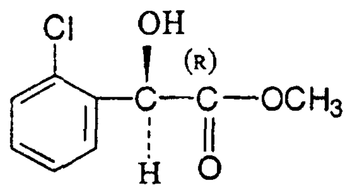

II

avec un anhydride ou halogénure de sulfonyle de formule générale :

$$R_1\text{-}SO_2\text{-}R_2 \qquad III$$

dans laquelle $R_2$ représente un groupement - $OSO_2$ - $R_1$ ou un atome d'halogène et $R_1$ a la même signification que dans la Revendication 1, la réaction ayant lieu en présence d'un sel de lithium et d'une amine aromatique, pour former le composé désiré.

**10.** Procédé selon la Revendication 9, **caractérisé en ce que** le sel de lithium est le perchlorate de lithium ou le tétrafluoroborate de lithium.

**11.** Procédé selon la Revendication 9 ou 10, **caractérisé en ce que** le sel de lithium est utilisé à raison de 1 à 1,2 équivalent molaire par équivalent molaire de composé de formule III.

**12.** Procédé selon une des Revendications 9 à 11, **caractérisé en ce que** l'amine aromatique est la pyridine ou la 4-diméthylaminopyridine.

**13.** Procédé selon la Revendication 12, **caractérisé en ce que** l'amine aromatique est utilisée à raison de 1 à 1,2 équivalent molaire par équivalent molaire de composé de formule III.

**14.** Procédé selon une des Revendications 9 à 13, **caractérisé en ce que** la réaction a lieu à une température comprise entre 0°C et la température ambiante.

**15.** Procédé de préparation d'esters (R)-sulfonyloxyacétiques selon une des Revendications 1 à 8, **caractérisé en ce que** l'on fait réagir l'ester (R)-2-(2-chloro-phényl)-2-hydroxy-acétique de formule:

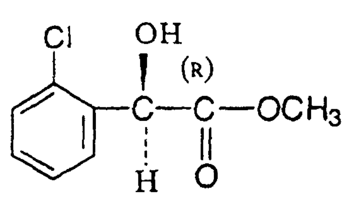

II

avec un anhydride ou halogénure de sulfonyle de formule générale :

$$R_1 - SO_2 - R_2 \qquad III$$

dans laquelle $R_2$ représente un groupement - $OSO_2$ - $R_1$ ou un atome d'halogène et $R_1$ a la même signification que dans la Revendication 1, la réaction ayant lieu en présence de 4-diméthylaminopyridine et d'un autre accepteur d'acide, pour former le composé désiré.

**16.** Procédé selon la Revendication 15, **caractérisé en ce que** l'accepteur d'acide est une amine.

**17.** Procédé selon la Revendication 15 ou 16, **caractérisé en ce que** la réaction a lieu à une température comprise entre - 10°C et + 10°C.

**18.** Procédé selon une des Revendications 9 à 17, **caractérisé en ce que** la réaction a lieu dans un solvant aprotique.

**19.** Procédé selon la Revendication 18, **caractérisé en ce que** le solvant aprotique est un hydrocarbure halogéné.

**20.** Procédé de préparation du clopidogrel de formule:

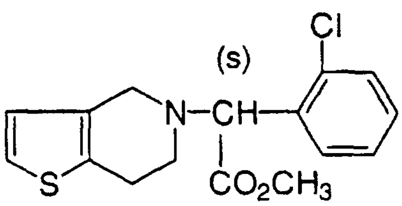

**caractérisé en ce que** l'on fait réagir la 4,5,6,7-tétrahydrothiéno [3,2-c] pyridine, sous forme de base ou de sel, avec un dérivé d'ester (R)-sulfonyloxyacétique selon une des Revendications 1 à 8 éventuellement en présence d'un agent basique utilisé seul ou en solution aqueuse, ce qui fournit le composé souhaité.

**21.** , Procédé selon la Revendication 20, **caractérisé en ce que** l'agent basique est un carbonate de métal alcalin ou un bicarbonate de métal alcalin.

**22.** Procédé selon la Revendication 20 ou 21, **caractérisé en ce que** la réaction a lieu à une température variant de la température ambiante à la température de reflux.

**23.** Procédé selon une des Revendications 20 à 22, **caractérisé en ce que** la réaction a lieu dans un solvant polaire ou non polaire.

**24.** Procédé selon la Revendication 23, **caractérisé en ce que** le solvant est un ester aliphatique en $C_2$-$C_5$, un alcool aliphatique en $C_1$-$C_4$, le N,N-diméthylformamide, un éther cyclique en $C_4$-$C_6$, un éther aliphatique en $C_2$-$C_6$, une cétone aliphatique en $C_2$-$C_8$, un hydrocarbure aliphatique halogéné en $C_1$-$C_4$ ou un hydrocarbure aromatique en $C_6$-$C_{10}$.

**25.** Procédé de préparation du clopidogrel de formule :

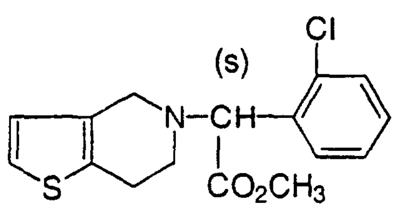

**caractérisé en ce que** :

a) l'on fait réagir la 2-(thién-2-yl)éthylamine sous forme de base ou de sel avec un dérivé d'ester (R)-sulfonyloxyacétique selon une des Revendications 1 à 8 en présence d'un agent basique utilisé seul ou en solution aqueuse, pour former le (+)-(S)-α-(2-(thién-2-yl)éthylamino)-α-(2-chlorophényl)-acétate de méthyle de

formule :

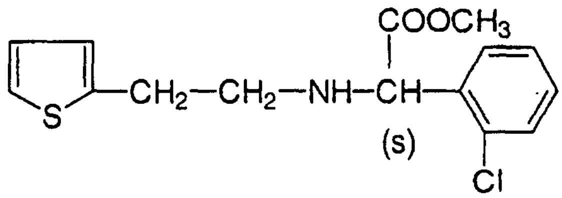

b) l'on fait réagir le dérivé de thiényléthylamine ainsi formé, avec un agent de formylation et on cyclise en présence d'un acide, ce qui fournit le composé souhaité.

**26.** Procédé selon la Revendication 25, **caractérisé en ce que** l'agent de formylation est choisi parmi l'aldéhyde formique, son hydrate ou ses dérivés de polymérisation, des composés de formule générale :

$$R_3 - CH_2 - R_4$$

dans laquelle $R_3$ représente un atome d'halogène, un groupement alkoxy en $C_1$-$C_4$, un groupement alkylthio en $C_1$ -$C_4$ ou un groupement amino et $R_4$ représente un groupement alkoxy en $C_1$-$C_4$, alkylthio en $C_1$-$C_4$, amino, alkoxycarbonyle en $C_2$-$C_5$ ou phénoxycarbonyle ou encore des composés hétérocycliques de formule générale:

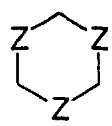

dans laquelle Z représente O, NH ou S.

**27.** Procédé selon la Revendication 26, **caractérisé en ce que** l'agent de formylation est l'aldéhyde formique, son hydrate ou ses dérivés de polymérisation.

**28.** Procédé selon une des Revendications 25 à 27, **caractérisé en ce que** l'acide est un acide minéral ou organique.

**29.** Procédé selon la Revendication 25, **caractérisé en ce que** la réaction avec le dérivé ester (R)-sulfonyloxyacétique a lieu dans un solvant polaire ou non-polaire.

**30.** Procédé selon la Revendication 25 , **caractérisé en ce que** la réaction avec l'agent de formylation et la cyclisation sont effectuées de manière simultanée dans un solvant polaire.

**31.** Procédé selon la Revendication 30, **caractérisé en ce que** l'acide est l'acide formique utilisé également comme solvant.

**Patentansprüche**

**1.** (R)-Sulfonyloxyessigsäureester-Derivate der allgemeinen Formel:

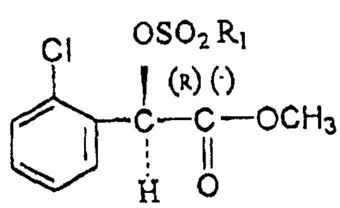

I

in der $R_1$ eine Benzylgruppe, eine $C_1$-$C_4$-Alkylgruppe, die gegebenenfalls durch ein oder mehrere Halogenatome substituiert ist, oder eine Phenylgruppe, die gegebenenfalls durch ein oder mehrere Halogenatome oder durch eine oder mehrere gradkettige oder verzweigte $C_1$-$C_4$-Alkylgruppen oder eine Nitrogruppe substitutert ist, bedeutet.

**2.** (R)-Sulfonyloxyessigsäureester-Derivate nach Anspruch 1, worin R1 eine Methyl-, Ethyl-, Propyl-, Trifluormethyl-, Benzyl-, Phenyl-, Chlorphenyl-, Tolyl-, Trimethylphenyl-, Triisopropylphenyl-, Dichlorphenyl- oder Nitrophenyl-Gruppe bedeutet.

**3.** (R)-2-Benzolsulfonyloxy-2-(2-chlorphenyl)-essigsäuremethylester.

**4.** (R)-2-(2-Chlorphenyl)-2-(2,4,6-trimethylbenzolsulfonyloxy)-essigsäuremethylester.

**5.** (R)-2-(4-Chlorbenzolsulfonyloxy)-2-(2-chlorphenyl)-essigsäuremethylester.

**6.** (R)-2-(2-Chlorphenyl)-2-(4-nitrobenzolsulfonyloxy)-essigsäuremethylester.

**7.** (R)-2-(2-Chlorphenyl)-2-(2,4,6-triisopropylbenzolsulfonyloxy)-essigsäuremethylester.

**8.** (R)-2-(2-Chlorphenyl)-2-(2,5-dichlorbenzolsulfonyloxy)-essigsäuremethylester.

**9.** Verfahren zur Herstellung der (R)-Sulfonyloxyessigsäureester nach einem der Ansprüche 1 bis 8, **dadurch gekennzeichnet, daß** man den (R)-2-(2-chlorphenyl)-2-hydroxyessigsäureester der Formel:

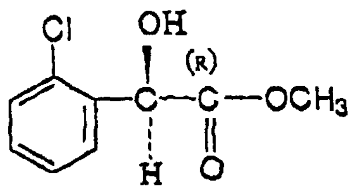

II

mit einem Sulfonyl-anhydrid oder -halogenid der allgemeinen Formel:

$$R_1 - SO_2 - R_2 \qquad III$$

in der $R_2$ eine Gruppe -$OSO_2$-$R_1$ oder ein Halogenatom darstellt und $R_1$ die in Anspruch 1 angegebenen Bedeutungen besitzt, unter Bildung der gewünschten Verbindung umsetzt, wobei die Reaktion in Gegenwart eines Lithiumsalzes und eines aromatischen Amins durchgeführt wird.

**10.** Verfahren nach Anspruch 9, **dadurch gekennzeichnet, daß** das Lithiumsalz Lithiumperchlorat oder Lithiumtetrafluorborat ist.

**11.** Verfahren nach den Ansprüchen 9 oder 10, **dadurch gekennzeichnet, daß** das Lithiumsalz in einer Menge von 1 bis 1,2 Moläquivalenten pro Moläquivalent der Verbindung der Formel III verwendet wird.

**12.** Verfahren nach einem der Ansprüche 9 bis 11, **dadurch gekennzeichnet, daß** das aromatische Amin Pyridin oder 4-Dimethylaminopyridin ist.

**13.** Verfahren nach Anspruch 12, **dadurch gekennzeichnet, daß** das aromatische Amin in einer Menge von 1 bis 1,2 Moläquivalenten pro Moläquivalent der Verbindung der Formel III verwendet wird.

**14.** Verfahren nach einem der Ansprüche 9 bis 13, **dadurch gekennzeichnet, daß** die Reaktion bei einer Temperatur zwischen 0°C und Raumtemperatur durchgeführt wird.

**15.** Verfahren zur Herstellung der (R)-Sulfonyloxyessigsäureester nach einem der Ansprüche 1 bis 8, **dadurch gekennzeichnet, daß** man den (R)-2-(2-Chlorphenyl)-2-hydroxyessigsäureester der Formel:

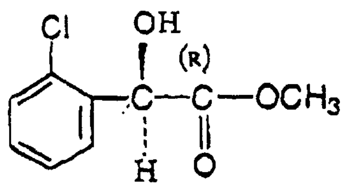

II

mit einem Sulfonyl-anhydrid oder -halogenid der allgemeinen Formel:

$$R_1 - SO_2 - R_2 \quad \text{III}$$

in der $R_2$ eine Gruppe $-OSO_2-R_1$ oder ein Halogenatom darstellt und $R_1$ die in Anspruch 1 angegebenen Bedeutungen besitzt, zur Bildung der gewünschten Verbindung umsetzt, wobei die Reaktion in Gegenwart von 4-Dimethylaminopyridin und eines anderen Säureakzeptors durchgeführt wird.

**16.** Verfahren nach Anspruch 15, **dadurch gekennzeichnet, daß** der Säureakzeptor ein Amin ist.

**17.** Verfahren nach Anspruch 15 oder 16, **dadurch gekennzeichnet, daß** die Reaktion bei einer Temperatur zwischen -10°C und +10°C durchgeführt wird.

**18.** Verfahren nach einem der Ansprüche 9 bis 17, **dadurch gekennzeichnet. daß** die Reaktion in einem aprotischen Lösungsmittel durchgeführt wird.

**19.** Verfahren nach Anspruch 18, **dadurch gekennzeichnet, daß** das aprotische Lösungsmittel ein halogenierter Kohlenwasserstoff ist.

**20.** Verfahren zur Herstellung von Clopidogrel der Formel:

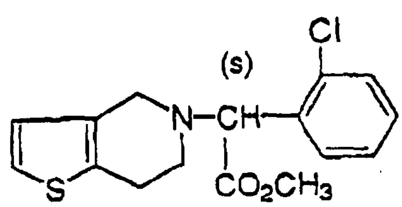

**dadurch gekennzeichnet, daß** man 4,5,6,7-Tetrahydrothieno[3,2-c]-pyridin in Form der Base oder des Salzes mit einem (R)-Sulfonyloxyessigsäureester-Derlvat nach einem der Ansprüche 1 bis 8 gegebenenfalls in Gegenwart eines basischen Mittels, welches allein oder in wässriger Lösung eingesetzt wird, zur Bildung der gewünschten Verbindung umsetzt.

**21.** Verfahren nach Anspruch 20, **dadurch gekennzeichnet, daß** das basische Mittel ein Alkalimetallcarbonat oder ein Alkalimetallbicarbonat ist.

**22.** Verfahren nach Anspruch 20 oder 21, **dadurch gekennzeichnet, daß** die Reaktion bei einer Temperatur von Raumtemperatur bis zu der Rückflußtemperatur durchgerührt wird.

**23.** Verfahren nach einem der Ansprüche 20 bis 22, **dadurch gekennzeichnet, daß** die Reaktion in einem polaren oder nicht-polaren Lösungsmittel durchgeführt wird.

**24.** Verfahren nach Anspruch 23, **dadurch gekennzeichnet, daß** das Lösungsmittel ein aliphatischer $C_2$-$C_5$-Ester, ein aliphatischer $C_1$-$C_4$-Alkohol, N,N-Dimethylformamid, ein cyclischer $C_4$-$C_6$-Ether, ein aliphatischer $C_2$-$C_6$-Ether, ein aliphatisches $C_2$-$C_8$-Keton, ein halogenierter aliphatischer $C_1$-$C_4$-Kohlenwasserstoff oder ein aromatischer $C_6$-$C_{10}$-Kohlenwasserstoff ist.

**25.** Verfahren zur Herstellung von Clopidogrel der Formel:

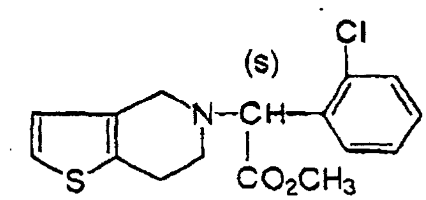

**dadurch gekennzeichnet, daß** man:

a) 2-(Thien-2-yl)-ethylamin in Form der Base oder des Salzes mit einem (R)-Sulfonyloxyesslgsäureester-Derivat nach einem der Ansprüche 1 bis 8 in Gegenwart des basischen Mittels, welches allein oder in wässriger Lösung eingesetzt wird, umsetzt zur Bildung von (+)-(S)-α-(2-(Thien-2-yl)-ethylamino)-α-(2-chlorphenyl)-essigsäureethylester der Formel:

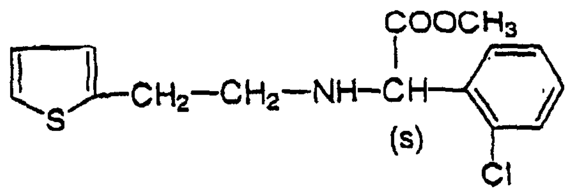

b) man das in dieser Weise gebildete Thienylethylamin-Derivat mit einem Formylierungsmittel umsetzt und in Gegenwart einer Säure cyclisiert zur Bildung der gewünschten Verbindung.

**26.** Verfahren nach Anspruch 25, **dadurch gekennzeichnet, daß** das Formylierungsmittel aus Formaldehyd, seinem Hydrat oder seinen Polymerisationsderivaten, Verbindungen der allgemeinen Formel:

$$R_3 - CH_2 - R_4$$

in der $R_3$ ein Halogenatom, eine $C_1$-$C_4$-Alkoxy-Gruppe, eine $C_1$-$C_4$-Alkylthio-Gruppe oder eine Aminogruppe und $R_4$ eine $C_1$-$C_4$-Alkoxy-Gruppe, eine $C_1$-$C_4$-Alkylthio-Gruppe, eine Aminogruppe, eine $C_2$-$C_5$-Alkoxycarbonyl-Gruppe oder Phenoxycarbonyl-Gruppe bedeuten, oder heterocyclischen Verbindungen der allgemeinen Formel:

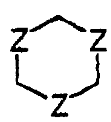

in der Z, O, NH oder S bedeutet, ausgewählt wird.

**27.** Verfahren nach Anspruch 26, **dadurch gekennzeichnet, daß** das Formylierungsmittel Formaldehyd, sein Hydrat oder eines seiner Polymerisationsderivate ist.

**28.** Verfahren nach einem der Ansprüche 25 bis 27, **dadurch gekennzeichnet, daß** die Säure eine anorganische oder organische Säure ist.

**29.** Verfahren nach Anspruch 25, **dadurch gekennzeichnet, daß** die Reaktion mit dem (R)-Sulfonyloxyessigsäureester-Derivat in einem polaren oder nicht-polaren Lösungsmittel durchgeführt wird.

**30.** Verfahren nach Anspruch 25, **dadurch gekennzeichnet, daß** die Reaktion mit dem Formylierungsmittel und die Cyclisierung gleichzeitig in einem polaren Lösungsmittel durchgeführt werden.

**31.** Verfahren nach Anspruch 30, **dadurch gekennzeichnet, daß** die Säure Ameisensäure ist, welche gleichzeitig als Lösungsmittel verwendet wird.

## Claims

**1.** (R)-Sulphonyloxyacetic ester derivatives of general formula:

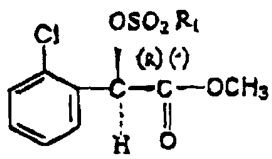

I

in which $R_1$ represents a benzyl group, a $C_1$-$C_4$ alkyl group optionally substituted by one or more halogen atoms, or a phenyl group optionally substituted by one or more halogen atoms or by one or more linear or branched $C_1$-$C_4$ alkyl groups or by a nitro group.

**2.** (R)-Sulphonyloxyacetic ester derivatives according to Claim 1, in which $R_1$ represents a methyl, ethyl, propyl, trifluoromethyl, benzyl, phenyl, chlorophenyl, tolyl, trimethylphenyl, triisopropylphenyl, dichlorophenyl or nitrophenyl group.

**3.** Methyl (R)-2-benzenesulphonyloxy-2-(2-chlorophenyl)acetate.

**4.** Methyl (R)-2-(2-chlorophenyl)-2-(2,4,6-trimethylbenzenesulphonyloxy) acetate.

**5.** Methyl (R)-2-(4-chlorobenzenesulphonyloxy)-2-(2-chlorophenyl)acetate.

**6.** Methyl (R)-2-(2-chlorophenyl)-2-(4-nitrobenzenesulphonyloxy)acetate.

**7.** Methyl (R)-2-(2-chlorophenyl)-2-(2,4,6-triisopropylbenzenesulphonyloxy)acetate.

**8.** Methyl (R)-2-(2-chlorophenyl)-2-(2,5-dichlorobenzenesulphonyloxy)acetate.

**9.** Process for the preparation of (R)-sulphonyloxyacetic esters according to one of Claims 1 to 8, **characterized in that** the (R)-2-(2-chlorophenyl)-2-hydroxyacetic ester of formula:

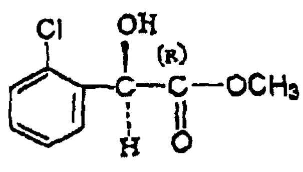

II

is reacted with a sulphonic anhydride or sulphonyl halide of general formula:

$$R_1\text{-}SO_2\text{-}R_2 \qquad III$$

in which $R_2$ represents an $-OSO_2-R_1$ group or a halogen atom and $R_1$ has the same meaning as in Claim 1, the reaction taking place in the presence of a lithium salt and of an aromatic amine, to form the desired compound.

**10.** Process according to Claim 9, **characterized in that** the lithium salt is lithium perchlorate or lithium tetrafluoroborate.

**11.** Process according to Claim 9 or 10, **characterized in that** the lithium salt is used in a proportion of 1 to 1.2 molar equivalents per molar equivalent of compound of formula III.

**12.** Process according to one of Claims 9 to 11, **characterized in that** the aromatic amine is pyridine or 4-dimethylaminopyridine.

**13.** Process according to Claim 12, **characterized in that** the aromatic amine is used in a proportion of 1 to 1.2 molar equivalents per molar equivalent of compound of formula III.

**14.** Process according to one of Claims 9 to 13, **characterized in that** the reaction takes place at a temperature between 0°C and ambient temperature.

**15.** Process for the preparation of (R)-sulphonyloxyacetic esters according to one of Claims 1 to 8, **characterized in that** the (R)-2-(2-chlorophenyl)-2-hydroxyacetic ester of formula:

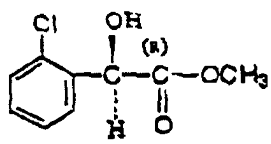

II

is reacted with a sulphonic anhydride or sulphonyl halide of general formula:

$$R_1\text{-}SO_2\text{-}R_2 \qquad \text{III}$$

in which $R_2$ represents an $-OSO_2-R_1$ group or a halogen atom and $R_1$ has the same meaning as in Claim 1, the reaction taking place in the presence of 4-dimethylaminopyridine and of another acid acceptor, to form the desired compound.

**16.** Process according to Claim 15, **characterized in that** the acid acceptor is an amine.

**17.** Process according to Claim 15 or 16, **characterized in that** the reaction takes place at a temperature between -10°C and +10°C.

**18.** Process according to one of Claims 9 to 17, **characterized in that** the reaction takes place in an aprotic solvent.

**19.** Process according to Claim 18, **characterized in that** the aprotic solvent is a halogenated hydrocarbon.

**20.** Process for the preparation of clopidogrel of formula:

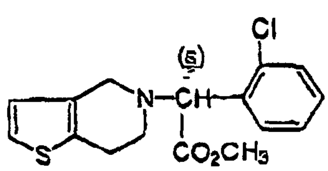

**characterized in that** 4,5,6,7-tetrahydrothieno[3,2-c]pyridine, in the base or salt form, is reacted with an (R)-sulphonyloxyacetic ester derivative according to one of Claims 1 to 8, optionally in the presence of a basic agent used alone or in aqueous solution, which provides the desired compound.

**21.** Process according to Claim 20, **characterized in that** the basic agent is an alkali metal carbonate or an alkali metal bicarbonate.

**22.** Process according to Claim 20 or 21, **characterized in that** the reaction takes place at a temperature varying from ambient temperature to the reflux temperature.

**23.** Process according to one of Claims 20 to 22, **characterized in that** the reaction takes place in a polar or nonpolar solvent.

**24.** Process according to Claim 23, **characterized in that** the solvent is a $C_2$-$C_5$ aliphatic ester, a $C_1$-$C_4$ aliphatic alcohol, N,N-dimethylformamide, a $C_4$-$C_6$ cyclic ether, a $C_2$-$C_6$ aliphatic ether, a $C_2$-$C_8$ aliphatic ketone, a halogenated $C_1$-$C_4$ aliphatic hydrocarbon or a $C_6$-$C_{10}$ aromatic hydrocarbon.

**25.** Process for the preparation of clopidogrel of formula:

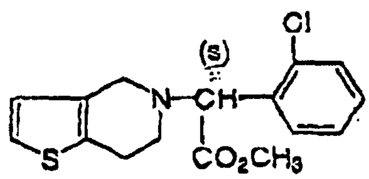

**characterized in that**:

a) 2-(thien-2-yl)ethylamine, in the base or salt form, is reacted with an (R)-sulphonyloxyacetic ester derivative according to one of Claims 1 to 8, in the presence of a basic agent used alone or in aqueous solution, to form methyl (+)-(S)-α-(2-(thien-2-yl)ethylamino)-α-(2-chlorophenyl)-acetate of formula:

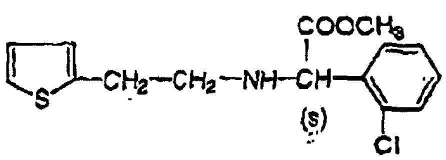

b) the thienylethylamine derivative thus formed is reacted with a formylating agent and cyclization is carried out in the presence of an acid, which provides the desired compound.

**26.** Process according to Claim 25, **characterized in that** the formylating agent is chosen from formaldehyde, its hydrate or its polymerization derivatives, compounds of general formula:

$$R_3\text{-}CH_2\text{-}R_4$$

in which $R_3$ represents a halogen atom, a $C_1$-$C_4$ alkoxy group, a $C_1$-$C_4$ alkylthio group or an amino group and $R_4$ represents a $C_1$-$C_4$ alkoxy, $C_1$-$C_4$ alkylthio, amino, $C_2$-$C_5$ alkoxycarbonyl or phenoxycarbonyl group, or heterocyclic compounds of general formula;

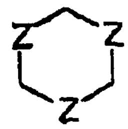

in which Z represents O, NH or S.

**27.** Process according to Claim 26, **characterized in that** the formylating agent is formaldehyde, its hydrate or its polymerization derivatives.

**28.** Process according to one of Claims 25 to 27, **characterized in that** the acid is an inorganic or organic acid.

**29.** Process according to Claim 25, **characterized in that** the reaction with the (R)-sulphonyloxyacetic ester derivative takes place in a polar or nonpolar solvent,

**30.** Process according to Claim 25, **characterized in that** the reaction with the formylating agent and the cyclization are carried out simultaneously in a polar solvent.

**31.** Process according to Claim 30, **characterized in that** the acid is formic acid, also used as solvent.